(19) Europäisches Patentamt
European Patent Office
Office européen des brevets

(11) EP 0 923 729 B1

# (12) EUROPEAN PATENT SPECIFICATION

(45) Date of publication and mention of the grant of the patent:
**12.12.2001 Bulletin 2001/50**

(21) Application number: **97935988.2**

(22) Date of filing: **25.08.1997**

(51) Int Cl.[7]: **G01N 33/00**, B01D 11/04

(86) International application number:
**PCT/SE97/01395**

(87) International publication number:
**WO 98/10281 (12.03.1998 Gazette 1998/10)**

(54) **USE OF A LIQUID CRYSTALLINE PHASE FOR DETERMINING THE DISTRIBUTION OF A CHEMICAL SUBSTANCE BETWEEN A HYDROPHOBIC AND A HYDROPHILIC ENVIRONMENT**

VERWENDUNG EINER FLÜSSIGEN KRISTALLINEN PHASE ZUR FESTSTELLUNG DER VERTEILUNG EINER CHEMISCHEN SUBSTANZ ZWISCHEN EINER HYDROPHOBEN UND EINER HYDROPHILEN UMGEBUNG

UTILISATION D'UNE PHASE CRISTALLINE LIQUIDE POUR DETERMINER LA REPARTITION D'UN PRODUIT CHIMIQUE ENTRE UN MILIEU HYDROPHOBE ET UN MILIEU HYDROPHILE

(84) Designated Contracting States:
**AT BE CH DE DK ES FI FR GB GR IE IT LI LU MC NL PT SE**

(30) Priority: **05.09.1996 SE 9603226**

(43) Date of publication of application:
**23.06.1999 Bulletin 1999/25**

(73) Proprietor: **GS DEVELOPMENT AB**
**213 75 Malmö (SE)**

(72) Inventors:
- **ENGSTRÖM, Sven**
  **22647 Lund (SE)**
- **WALLIN, Ronnie**
  **S-233 39 Svedala (SE)**
- **PETERSSON NORDEN, Tomas**
  **S-151 73 Södertälje (SE)**

(74) Representative: **Petri, Stellan et al**
**Ström & Gulliksson AB**
**Box 41 88**
**203 13 Malmö (SE)**

(56) References cited:
**EP-A- 0 420 683**
**WO-A-95/34287**

- **PROCEED. INTERN. SYMP. CONTROL. REL. BIOACT. MATER., Volume 23, 1996, T. PETTERSSON et al., "Interaction of Clomethiazole with Model Membranes", pages 467-468.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 923 729 B1

## Description

### Technical Field

The invention refers to the use of a liquid crystalline phase for determining the distribution of a chemical substance between a hydrophobic and a hydrophilic environment. More precisely, the invention refers to the use of a cubic liquid crystalline phase for determining the distribution of a chemical substance between a hydrophobic and a hydrophilic environment, the hydrophobic environment being represented by a lamellar bilayer of lipids in the cubic liquid crystalline phase and the hydrophilic environment by a hydrophilic phase in equilibrium with the cubic phase.

### Prior Art

It is a well known fact that oil and water are not inter-mixable. In this connection oil means molecules composed of a considerable portion of hydrocarbon, for example alkanes, fatty alcohols and triglycerides. Another group of substances is the polar lipids which apart from hydrocarbons also comprise water soluble groups in the same molecule. In nature these substances play an important role as components of cell membranes. Thus, they represent a natural wall between two environments which both can be aqueous.

In a typical case the active substance in a drug must pass several such barriers. Accordingly, it is alternately located in an aqueous and in a hydrocarbon environment. The property of the active substance with respect to its solubility in one environment or the other can thus be of great importance for its ability to accomplish the effect intended on the organism.

A traditional way of measuring the preference of a chemical substance for one or the other of a hydrophobic environment or an hydrophilic environment is to add the substance in question to a system comprising octanol and water (see e.g. Leo et al. (1971) Chem. Rev. 71:525-616). Octanol is an alcohol with a very low solubility in water and may represent the hydrophobic environment. When an equilibrium has been reached the concentration of the substance in question can be determined in the octanol phase as well as in the water phase. The ratio obtained between the concentrations,

$[\text{substance}]_{octanol}/[\text{substance}]_{water}$,

is usually called the distribution coefficient and is represented by $K_{octanol/water}$.

A substance with a preference for the octanol phase will result in a value of $K_{octanol/water}$ which is higher than 1 whereas a substance with a preference for water results in a value which is lower than 1. An advantage of this method is that the procedure is simple and the substances used in the method are cheap.

However, a problem with the system octanol/water is that it poorly imitates the conditions prevailing in living organisms, the hydrophobic environment often comprising a cell membrane and only in exceptional cases a hydrocarbon in bulk phase. The cell membrane structure comprises what is called a lamellar bilayer of polar lipids, and in most cases the interior of the membrane can be resembled to the conditions of a liquid hydrocarbon. Thus, an alternative to the system octanol/water would be to use a system, in which the hydrophobic environment is represented by a lamellar bilayer of polar lipids.

Lamellar bilayers can either be obtained from cell membranes, e.g. erythrocytes, or as artificially produced vesicles of membrane lipids (see e.g. Pauleti & Wunderli-Allenspach (1994) Eur. J. Pharm. Sci. 1:273-282). A property shared by these systems is that their use when determining the distribution of a substance comprises complex preparation and separation steps in comparison with the system octanol/water. This makes the membrane systems less suitable for routine analysis. However, the distribution coefficients determined by means of the membrane systems have turned out to present interesting information which supplements the results obtained from the system octanol/water. This fully motivates the use of an alternative to the latter.

The article by T. Petersson et al. (Proceed. Intern. Symp. Control. Rel. Bioact. Mater., 23 (1996) pp. 467-468) concerns the determination of the hydrophilic/lipophilic properties of the drug clomethiazole (CMZ) in different systems, one of these being a lipid laminar bilayer cubic phase of glycerolmonooleate in 30% w/w water. The interaction between a cubic liquid crystalline phase of glycerolmonooleate and CMZ was studied in order to obtain information which is not predictable from the octanol/water or a similar partition coefficient.

In EP-A-0 420 683 a method is shown for the evaluation of drug activity and structure. The lipid film-water partition coefficient of a chemical substance was determined by measurements based on changes in oscillation frequence of a crystal oscillator, which result from the interaction between the substance and a lipid bilayer.

A biologically active composition containing diacylglycerol and phospholipid is shown in WO-A-95/34287. A cubic liquid crystalline phase is formed for encapsulating a biologically active material for controlled release of the same.

**Description of the Invention**

The present invention satisfies the demand for a system, which better represents the hydrophobic environments in living organisms as well as the demand for easy handling, and concerns the use of a cubic liquid crystalline phase for determining the distribution of a chemical substance between a hydrophobic and a hydrophilic environment. According to the invention the hydrophobic environment comprises a lamellar bilayer of lipids in the cubic liquid crystalline phase. The hydrophilic environment is represented by a hydrophilic phase in equilibrium with the cubic phase.

The use according to the invention is of great advantage since the lipids in the cubic phase of very high viscosity can be produced from a broad spectrum of biologically relevant lipids. Such lipids can be biological lipids, semisynthetic lipids, synthetic lipids, or mixtures thereof. Examples of biological lipids in the cubic phase are monoglycerides, phospholipids, glycolipids, sphingolipids, or mixtures thereof. However, they preferably comprise monoglycerides. Examples of monoglycerides are glyceryl monooleate, glyceryl monolinoleate, and glyceryl monolinolenate, and mixtures of these can also be used. A mixture of monoglycerides and phospholipids can also be utilized when the cubic phase is used according to the invention, a mixture of diacylphosphatidylcholine and diacylphosphatidylethanolamine being preferred.

On a molecular level the inner structure of the cubic phase results in resemblance to the existing membrane structures in cells and vesicles. Furthermore, the solid consistency of the cubic phase results in that it is very easy to separate from a surrounding water solution. Since several types of biologically relevant lipids can be used for producing the cubic phase the options are considerable to create systems which are adapted for special purposes, e.g. to imitate different kinds of barriers in a living organism.

Another advantage of the system is that it can be used for automatic determinations since it is very easy to separate the water phase from the cubic phase due to the rigidity of the cubic phase. When a not finely divided cubic phase is used a water phase only has to be decanted, i.e. a test vial has only to be turned up and down in order to separate the water phase from the cubic phase in the vial. The two phases can then be analyzed separately with reference to their contents. This method is especially adaptable in those cases when radiolabelled substances are used, e.g. in scintigraphic methods, which can be highly automated. Within the field of liquid chromatography a special sampling device usually sucks up a sample from a test vial. The present system can also be used in these cases since the rigidity of the cubic phase results in that a sample can be taken from the water phase without the cubic phase interfering with the sampling procedure.

An important aspect in connection with analysis is the time required for the experiment in question. The present system is based on that the equilibrium is to be reached. The time taken for this depends above all on the system used, its dimensions as well as that surface which the cubic phase exposes to the water phase. The dimensions of the system depend not only on the desired accuracy when determining the distribution coefficient but also on the accuracy of the analytical method. The surface the cubic phase exposes to the water phase is determined by the geometry of the test vial. A method of increasing the area is to finely divide the cubic phase by utilizing some form of inert carrier in the form of a particle. One example is to use porous spheres of cellulose. However, the invention does not exclude other carrier materials as long as they do not influence the formation of the cubic phase. This is controlled by means of X-ray diffraction, nuclear magnetic resonance spectroscopy, or by both methods. If the cubic phase is formed in and/or on a particle shaped carrier these particles can be packed in a column for the determination of the distribution, a water phase in this case being used as mobile phase. The method according to the invention will then obtain the character of a chromatographic method. The invention also comprises the possibility that the cubic phase is allowed to be formed on the walls of the column. The cubic phase in equilibrium with an hydrophilic phase can thus according to the invention be in the form of a homogenous phase, a finely divided phase in the form of discrete particles, or a mixture thereof.

The determination of the distribution of a chemical substance between a hydrophobic environment and a hydrophilic environment is based on that the lipid structure is not affected to any great extent. However, if the concentration becomes too high the structure of the cubic phase can be influenced in such a way that another phase is formed. The knowledge of the structure of this phase as well as the concentration of the chemical substance required for its formation can supply further information to interested persons. Another possibility is to follow the formation of the equilibrium with respect to time. The information obtained from such experiments can give further information about the transport routes for the chemical substance in the system. The experiments can also be used for establishing the optimal conditions required for analytical accuracy and for determining the distribution coefficient with reference to the time required for equilibrium formation.

In summary, from a handling point of view the present invention represents an excellent system for determining the distribution of a substance between a hydrophobic environment in the form of a lamellar bilayer of lipids in the cubic liquid crystalline phase and a hydrophilic environment. The preparation and separation steps are drastically reduced in complexity while the system at the same time provides for a production of test vials which are ready to be filled, the vials being in the form of containers suitable for automated analytical methods existing on the market. Liquid chromatography and liquid scintillation are examples of such methods. The possibility to pack chromatographic columns with the present system is a further example of the versatility of the system. For the analytical personnel the present invention also provides for a convenient method to measure the distribution of a chemical substance between a hydrophobic environment and a hydrophilic environment without time consuming and complicated preparation and separation steps. The present invention implies that the person evaluating the results from the measurement now has a system available which more closely resembles the existing conditions in a biological system.

**Examples**

*Example 1. Description of a general embodiment of the invention.*

Case 1 - Water soluble substances.

The present invention is based on that the lamellar bilayers in the cubic phase are spontaneously formed in contact with a water solution. On the other hand, vesicles and liposomes are produced by mechanical energy being supplied. The cubic phase formed is characterized by means of X-ray diffraction and nuclear magnetic resonance spectroscopy. These methods provide information regarding whether the cubic phase is constructed from a lamellar bilayer or not as well as its dimensions.

The distribution of a substance X between lipids and water in the system is depicted by the following distribution coefficient.

$$K_{bilayer/water} = K_{bl/w} = [X]_{lipid}/[X]_{water} \qquad (1),$$

wherein $[X]_{lipid}/[X]_{water}$ is the concentration of a substance X in the lipid and the water portion of the system, respectively. In order to be able to determine $K_{bl/w}$ several methods can be applied. If $v_{lipid}$ is the lipid volume, $v_1$ is the volume of water solution without the substance X, and $v_2$ is the volume of water solution with the substance X in a concentration of $[X]_0$ mol/litre, the distribution coefficient can be expressed as

$$K_{bl/w} = \{v_2\ ([X]_0-[X]_w)/[X]_w - v_1\}/v_{lipid} \qquad (2),$$

in which $[X]_w$ is the concentration of X in the water phase at equilibrium. Equation 2 can be used if only the water phase and not the lipid phase is analyzed with reference to its content of X.

If both the water phase and the cubic phase are analyzed with reference to their content of the substance X the following distribution coefficient can be determined

$$K_{cubic\ phase/water} = K_{Q/w} = [X]_Q/[X]_w \qquad (3),$$

in which $[X]_Q$ is the concentration of X in the cubic phase. A relationship between $K_{bl/w}$ and $K_{Q/w}$ can be derived if the volumetric portion of water in the cubic phase as well as the concentration of X in the water domain of the cubic phase are known. The former can be determined by means of X-ray diffraction. If it is assumed that the concentration of X in the water domain in the cubic phase is $[X]_w$, i.e. the same as in the water phase, the following relationship can be obtained

$$K_{bl/w} = \{K_{Q/w} - f_w\}/\{1-f_w\} \qquad (4)$$

in which $f_w$ is a volumetric portion of water in the cubic phase.

Case 2 - Substances sparingly soluble in water.

In this case it might be more appropriate to mix a known amount of the substance in question with a known volume of a mixture of polar lipids in order to dissolve the substance in the lipids before the addition of water, $V_{aq}$, and before the formation of the cubic phase. The calculation of the distribution coefficient can be made with the following formula

$$K_{bl/w} = [X]_0/[X]_w - V_{aq}/V_{lipid} \quad (5).$$

*Example 2. Determination of distribution coefficients for clomethiazole.*

Clomethiazole (CMZ) is a drug which at a low pH exists in a charged state and at a high pH in a neutral stage. Glyceryl monooleate (Grindsted/Danisco A/S, Brabrand, Denmark), 0.74 ml, was added to each of six vials. Then 0.3 ml water was added to three of the vials, and 0.3 ml citrate buffer, pH 3.4, was added to the remaining three vials. The cubic phase was allowed to be formed overnight. Then 1.0 ml of solutions with different concentrations of CMZ were added to each of the vials.

The contents were analyzed after 120 h with reference to CMZ in the water phase as well as the cubic phase. In this example the constants were $v_{lipid}$=0.74 ml, $v_1$=0.3 ml, $v_2$=1.0 ml, and $f_w$=0.4. The distribution coefficients $K_{bl/w}$ were calculated by means of the equations (2) and (4). The parameters $[X]_w$ and $K_{bl/w}$ in the three systems (I, II, and III) were found to be as shown below

| | I | II | III |
|---|---|---|---|
| $[X]_0$/mg/ml | 0.28 | 1.5 | 3.0 |
| water, $[X]_w$/mg/ml | 0.0072 | 0.038 | 0.076 |
| water, $K_{bl/w}$, eq. (2) | 57.0 | 57.9 | 57.9 |
| water, $[X]_Q$/mg/ml | 0.243 | 1.286 | 2.463 |
| water, $K_{bl/w}$, eq.(4) | 55.6 | 55.7 | 53.3 |
| citrate, $[X]_w$/mg/ml | 0.014 | 0.067 | 0.136 |
| citrate, $K_{bl/w}$ eq. (2) | 28.4 | 32.0 | 31.5 |
| citrate, $[X]_w$/mg/ml | 0.243 | 1.188 | 2.366 |
| citrate, $K_{bl/w}$, eq. (4) | 28.2 | 28.9 | 28.3 |

The table shows that the results obtained are relatively insensible to the CMZ content in the original solution.

In the case when only the CMZ content in the water solution is known the determination of the distribution coefficient can be made with reasonable accuracy. If the charged form of CMZ mainly appears in the citrate system it is also reasonable that the distribution coefficient in this case is lower than in water.

*Example 3. Determination of the distribution coefficient for lidocaine by the simultaneous addition of all the water solution.*

Lidocaine is a local anesthetic which at a low pH exists in a charged state and at a high pH in a neutral state. Glyceryl monooleate, 2 ml, was weighed into three vials, and 2.5 ml of a buffer solution of pH 4, 7 or 10, containing 1.00 lidocaine per millilitre was then added to either of the three vials. Then 0.5 ml of the respective buffer solution was added to the vials.

After 24 h the contents in the water phases were spectrophotometrically (262 nm) analyzed with reference to lidocaine. In this example the constants were $v_{lipid}$=2 ml, $v_1$=0.5 ml, $v_2$=2.5 ml and $[X]_0$=1.00 mg/ml. The distribution coefficient $K_{bl/w}$ was calculated by means of equation 2. The parameters $[X]_w$ and $K_{bl/w}$ in the three systems (I, II, and III) were found to be

| | I | II | III |
|---|---|---|---|
| pH | 4 | 7 | 10 |
| $[X]_w$/mg/ml | 0.68 | 0.21 | 0.073 |
| $K_{bl/w}$ | 0.7 | 8.9 | 31.2 |

Since $pK_a$ for lidocaine is 8 its charged form dominates at pH 4 while the uncharged form dominates at pH 10. Thus, $K_{bl/w}$(charged) should be 0.7 and $K_{bl/w}$(uncharged) should be 31.2.

*Example 4. Determination of the distribution coefficient for lidocaine by the addition of a water solution after the cubic phase has been formed.*

Glyceryl monooleate, 2 ml, was weighed into three vials, and 0.5 ml of a buffer solution of pH 4, 7 or 10 was then added to one of the three vials. The cubic phase was allowed to be formed for 24 h at 37 °C in an incubator. Then 2.5 ml of the same buffer solution containing 1.00 mg lidocaine per millilitre was added to the respective test vial.

After further 24 h the contents in the water phase were spectrophotometrically (262 nm) analyzed with reference to lidocaine. In this example the constants were $v_{lipid}$=2 ml, $v_1$=0.5 ml, $v_2$=2.5 ml, and $[X]_0$=1.00 mg/ml. The distribution coefficient $K_{bl/w}$ was calculated by means of equation 2. The parameters $[X]_w$ and $K_{bl/w}$ in the three systems (I, II, and III) were found to be

| | I | II | III |
|---|---|---|---|
| pH | 4 | 7 | 10 |
| $[X]_w$/mg/ml | 0.66 | 0.22 | 0.073 |
| $K_{bl/w}$ | 0.8 | 8.4 | 31.2 |

Since the present invention utilizes the equilibrium properties of the system the consecutive order of the additives will not matter as long as equilibrium prevails in the system. The similarity obtained when the results from example 3 and 4 are compared demonstrates this with no room for doubt.

*Example 5. Determination of the distribution coefficient for NaCl.*

Glyceryl monooleate, 2 ml, *was* weighed into three vials, 6 ml of a water solution with 0.033, 0.067, or 0.10 mol NaCl per litre then being added to one of the three vials.

After 72 h the NaCl contents in the three vials were determined by means of conductometrical analysis of the water phases. In this example the constants were $v_{lipid}$= 2 ml, $v_1$=0 ml, and $v_2$=6 ml. The distribution coefficient $K_{bl/w}$ was calculated by means of equation 2. The parameters $[X]_w$ and $K_{bl/w}$ in the three systems (I, II, and III) were found to be

| | I | II | III |
|---|---|---|---|
| $[X]_0$/mol/liter | 0.033 | 0.067 | 0.100 |
| $[X]_w$/mol/liter | 0.036 | 0.071 | 0.106 |
| $K_{bl/w}$ | -0.25 | -0.17 | -0.17 |

The negative distribution coefficients obtained in this case demonstrate that NaCl avoids the water domain of the cubic phase. This results in an increase in the salt concentration increases in the water phase in equilibrium with the cubic phase. A negative distribution coefficient is obtained since the equation 2 is based on an assumption that the concentration of the substance in question is similar in all the water of the system. It can thus be concluded that relatively small ions, such as $Na^+$ and $Cl^-$, avoids water domains in the vicinity of lipids while large ions, such as the charged form of lidocaine (example 3 and 4) have a certain affinity for the lipid system.

*Example 6. Determination of the distribution coefficients for alfentanil, fentanyl, and sufentanil by the simultaneous addition of all the water solution.*

Alfentanil, fentanyl and sufentanil belong to the group called general anesthetics, which at a low pH exist in a charged state and at a high pH in a neutral state. The dissociation constants for the three substances are 6.5, 8.4, and 8.0, respectively. Each drug in 0.9 ml buffer solution of pH 4, 7, or 10 was added to the test vials.

After 96 h the contents of the water phases were analyzed with reference to the drug by means of high performance liquid chromatography (HPLC). In this example the constants were $v_{lipid}$=0.3 ml, $v_1$=0 ml and $v_2$=0.9 ml. The distribution coefficients $K_{bl/w}$ were calculated by means of equation 2. The parameters $[X]_w$ and $K_{bl/w}$ in the three systems were found to be

| Alfentanil: | | | |
|---|---|---|---|
| | I | II | III |
| pH | 4.09 | 7.33 | 9.66 |
| $[X]_0$/mg/litre | 0.500 | 0.500 | 0.500 |
| $[X]_w$/mg/ml | 0.203 | 0.0148 | 0.0180 |
| $K_{bl/w}$ | 4.4 | 98 | 80 |

(continued)

| Fentanyl: | | | |
|---|---|---|---|
| | I | II | III |
| pH<br>$[X]_0$/mg/litre<br>$[X]_w$/mg/ml<br>$K_{bl/w}$ | 4.09<br>0.200<br>0.0422<br>11.2 | 7.33<br>0.200<br>0.0056<br>104 | 9.66<br>0.200<br>0.00025<br>2397 |
| Sufentanil: | | | |
| | I | II | |
| pH<br>$[X]_0$/mg/litre<br>$[X]_w$/mg/ml<br>$K_{bl/w}$ | 4.0<br>0.100<br>0.0133<br>19.6 | 6.0<br>0.100<br>0.00535<br>53 | |

For the three substances alfentanil, fentanyl and sufentanil the distribution coefficients in the system octanol/water were found to be 128, 813, and 1778, respectively. The results obtained above demonstrate with no room for doubt that this relative order of the substances also prevails in the present system.

## Claims

1. Use of a cubic liquid crystalline phase for determining the distribution of a chemical substance between a hydrophobic and a hydrophilic environment, the hydrophobic environment being represented by a lamellar bilayer of lipids in the cubic liquid crystalline phase and the hydrophilic environment by a hydrophilic phase in equilibrium with the cubic phase.

2. Use as claimed in claim 1, **characterized in that** the lipids in the cubic phase comprise biological lipids, semisynthetic lipids, synthetic lipids, or mixtures thereof.

3. Use as claimed in claim 2, **characterized in that** the biological lipids in the cubic phase comprise monoglycerides, phospholipids, glycolipids, sphingolipids, or mixtures thereof.

4. Use as claimed in claim 3, **characterized in that** the biological lipids comprise monoglycerides.

5. Use as claimed in claim 4, **characterized in that** the monoglycerides comprise glyceryl monooleate, glyceryl monolinoleate, glyceryl monolinolenate, or mixtures thereof.

6. Use as claimed in claim 3, **characterized in that** the lipids in the cubic phase comprise a mixture of monoglycerides and phospholipids.

7. Use as claimed in claim 3, **characterized in that** the lipids in the cubic phase comprise a mixture of diacylphosphatidylcholine and diacylphosphatidylethanolamine.

8. Use as claimed in claim 1, **characterized in that** the cubic phase in equilibrium with a hydrophilic phase is in the form of a homogeneous phase, a finely divided phase in the form of discrete particles, or a mixture thereof.

## Patentansprüche

1. Verwendung einer kubischen flüssigkristallinen Phase zur Bestimmung der Verteilung einer Chemikalie zwischen einer hydrophoben und einer hydrophilen Umgebung, wobei die hydrophobe Umgebung durch eine lamellenartige Lipiddoppelschicht in der kubischen flüssigkristallinen Phase und die hydrophile Umgebung durch eine hydrophile Phase im Gleichgewicht mit der kubischen Phase dargestellt wird.

2. Verwendung nach Anspruch 1, **dadurch gekennzeichnet, daß** die Lipide in der kubischen Phase biologische Lipide, halbsynthetische Lipide, synthetische Lipide oder deren Mischungen umfassen.

3. Verwendung nach Anspruch 2, **dadurch gekennzeichnet, daß** die biologischen Lipide in der kubischen Phase Monoglyceride, Phospholipide, Glycolipide, Sphingolipide oder deren Mischungen umfassen.

4. Verwendung nach Anspruch 3, **dadurch gekennzeichnet, daß** die biologischen Lipide Monoglyceride umfassen.

5. Verwendung nach Anspruch 4, **dadurch gekennzeichnet, daß** die Monoglyceride Glycerylmonooleat, Glycerylmonolinoleat, Glycerylmonolinolenat oder deren Mischungen umfassen.

6. Verwendung nach Anspruch 3, **dadurch gekennzeichnet, daß** die Lipide in der kubischen Phase eine Mischung aus Monoglyceriden und Phospholipiden umfassen.

7. Verwendung nach Anspruch 3, **dadurch gekennzeichnet, daß** die Lipide in der kubischen Phase eine Mischung aus Diacylphosphatidylcholin und Diacylphosphatidylethanolamin umfassen.

8. Verwendung nach Anspruch 1, **dadurch gekennzeichnet, daß** die kubische Phase, die sich im Gleichgewicht mit einer hydrophilen Phase befindet, in Form einer homogenen Phase, einer feinverteilten Phase in Form diskreter Partikel oder einer Mischung davon vorliegt.

**Revendications**

1. Utilisation d'une phase cristalline liquide cubique pour déterminer la répartition d'une substance chimique entre un milieu hydrophobe et un milieu hydrophile, l'environnement hydrophobe étant représenté par deux couches lamellaires de lipides dans la phase cristalline liquide cubique et l'environnement hydrophile par une phase hydrophile en équilibre avec la phase cubique.

2. Utilisation selon la revendication 1, **caractérisée en ce que** les lipides dans la phase cubique comprennent des lipides biologiques, des lipides semi-synthétiques, des lipides synthétiques, ou des mélanges de ceux-ci.

3. Utilisation selon la revendication 2, **caractérisée en ce que** les lipides biologiques dans la phase cubique comprennent des monoglycérides, des phospholipides, des glycolipides, des sphingolipides, ou des mélanges de ceux-ci.

4. Utilisation selon la revendication 3, **caractérisée en ce que** les lipides biologiques comprennent des monoglycérides.

5. Utilisation selon la revendication 4, **caractérisée en ce que** les monoglycérides comprennent le monooléate de glycéryle, le monolinoléate de glycéryle, le monolinolénate de glycéryle, ou des mélanges de ceux-ci.

6. Utilisation selon la revendication 3, **caractérisée en ce que** les lipides dans la phase cubique comprennent un mélange de monoglycérides et de phospholipides.

7. Utilisation selon la revendication 3, **caractérisée en ce que** les lipides dans la phase cubique comprennent un mélange de diacylphosphatidylcholine et de diacylphosphatidyléthanolamine.

8. Utilisation selon la revendication 1, **caractérisée en ce que** la phase cubique en équilibre avec une phase hydrophile se présente sous la forme d'une phase homogène, d'une phase finement divisée sous la forme de particules séparées, ou d'un mélange de celles-ci.